(19) Europäisches Patentamt
European Patent Office
Office européen des brevets

(11) **EP 2 851 028 A1**

(12) **EUROPÄISCHE PATENTANMELDUNG**

(43) Veröffentlichungstag:
25.03.2015 Patentblatt 2015/13

(51) Int Cl.:
*A61B 18/14* (2006.01)   *A61B 19/00* (2006.01)

(21) Anmeldenummer: **14177423.2**

(22) Anmeldetag: **17.07.2014**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **15.08.2013   US 201361866052 P**

(71) Anmelder: **VascoMed GmbH**
**79589 Binzen (DE)**

(72) Erfinder: **Bitzer, Andreas**
**8032 Zürich (CH)**

(74) Vertreter: **Lindner-Vogt, Karin L.**
**Biotronik SE & Co. KG**
**Corporate Intellectual Properties**
**Woermannkehre 1**
**12359 Berlin (DE)**

(54) **Katheteranordnung und Verfahren zur Bestimmung einer auf ein Katheterende einwirkenden Kraft**

(57)   Katheteranordnung, welche einen Katheter mit einem proximalen und einem distalen Ende und mindestens einem durchgehenden Fluidkanal, an dessen proximalem Ende ein Fluidanschluss und an dessen distalem Ende eine Düse angeordnet ist, die einen durch eine auf das distale Katheterende einwirkende Kraft veränderlichen Querschnitt hat, eine mit dem proximalen Fluidanschluss des Katheters verbundene Flüssigkeitszuführeinheit zur Zuführung von Flüssigkeit in den oder mindestens einen Fluidkanal des Katheters unter vorbestimmten Betriebsdruck, wobei in der Flüssigkeitszuführeinheit ein Flusssensor zur Erfassung eines Druckabfalls am von der Flüssigkeit durchströmten Fluidkanal im Betrieb der Katheteranordnung vorgesehen ist, und eine eingangsseitig signalmäßig mit dem Flusssensor verbundene Druckauswertungseinheit zur Bestimmung einer auf das distale Katheterende des Katheters einwirkenden Kraft aus dem erfassten Druckabfall am Fluidkanal und vorbestimmten Verformungscharakteristika der zugeordneten Düse umfasst.

FIG. 1

EP 2 851 028 A1

**Beschreibung**

Die Erfindung betrifft eine Katheteranordnung, die einen Katheter mit einem proximalen und einem distalen Ende und mindestens einem durchgehenden Fluidkanal, an dessen proximalen Ende ein Fluidanschluss angeordnet ist, und eine mit diesem Fluidanschluss verbundene Flüssigkeitszuführeinheit umfasst. Sie betrifft des Weiteren ein Verfahren zur Bestimmung einer auf das distale Ende eines entsprechenden Katheters einwirkenden Kraft.

In bestimmten Einsatzfeldern von Kathetern oder ähnlichen Vorrichtungen, z.B. Elektrodenleitungen, ist eine Andruckkraft an benachbartes Gewebe von Bedeutung für deren Funktion, sodass eine Erfassung dieser Kontaktkraft von Interesse ist. Dies trifft in besonderem Maße für sogenannte Ablationskatheter zu, mit denen Gewebebereiche verödet bzw. Gewebeteile abgetragen werden.

Es ist ein Ablationskatheter ("TactiCath", Fa. Endosense) bekannt, welches die Messung einer auf das distale Katheterende einwirkenden Kraft - im Anwendungsfall also der erwähnten Kontaktkraft - nach Betrag und Richtung während eines Ablationsvorganges ermöglicht. Dieser Katheter nutzt das Prinzip des sogenannten FBG(Faser-Bragg-Gitter)-Sensors, wobei drei Fasern mit jeweils einem FBG-Sensor am Faserende die für eine 3D-Kraftmessung benötigte Gruppe von Sensoren bilden, die zur gemeinsamen Messsignalverarbeitung an eine Signalverarbeitungseinheit einschließbar sind. Die Sensoren sind in einem Winkelabstand von 120° außen auf einem verformbaren Zylinder aufgebracht.

In der US 2008/0285909 A1 wird die Funktionsweise von FBG-Sensoren zur Bestimmung von Verdrillungen oder Krümmungen des Katheterkörpers ausführlich beschrieben, und auch die Funktionsweise des vorgenannten Kraftsensors mit mehreren FBG-Fasern auf einem verformbaren Zylinder ist in dieser Druckschrift erläutert.

Es ist, wie in der WO 2009/138957 A2 beschrieben, eine Temperaturkompensation mittels dreier elektrischer Thermoelemente vorgesehen, weil bei dem FBG-Messverfahren bereits kleine Temperaturänderungen bzw. Abweichungen zwischen den einzelnen Sensoren große Messunsicherheiten verursachen können und bei einem elektrothermischen Ablationsvorgang ganz erhebliche Temperaturschwankungen an der Spitze des Ablationskatheters auftreten können.

Das optische Messprinzip der FBG-Sensorik ist allgemein sowie insbesondere auch in seiner Anwendung für Kraftmessungen und Temperaturmessungen bekannt; vgl. etwa www.wikipedia.org/wiki/Fiber_Bragg_grating oder A. Othonos, K. Kalli:"Fiber Bragg Gratings: Fundamentels and Applications in Telecommunications and Sensing" Artec House 1999, sowie (speziell bezogen auf Spannungs- und Temperaturmessungen) US 5,399,854. Eine ausführliche Erläuterung des Messprinzips ist daher hier nicht erforderlich.

Unabhängig von diesem Messprinzip sind auch andere Lösungen für eine Kontaktkraftmessung an einem Führungsdraht oder Katheter bekannt, bspw. unter Einsatz eines optischen Sensors, wie in der WO 2009/007857 A2 beschrieben, oder unter Einsatz eines Halbleitersensors an der Spitze eines Führungsdrahtes, wie in der WO 2008/003307 A2 beschrieben.

Jüngere Verbesserungen der vorgenannten Lösungen sind Gegenstand der auf die Anmelderin zurückgehenden US-Anmeldung 61/446,053 und 61/703,272.

Der Erfindung liegt die Aufgabe zugrunde, eine in ihrem Aufbau als vereinfachte und daher kostengünstige Katheteranordnung der eingangs genannten Art anzugeben. Des Weiteren soll ein hinreichend verlässliches und mit einem einfachen kostengünstigen Aufbau realisierbares Messverfahren der o. g. Art bereitgestellt werden.

Diese Aufgabe wird in ihrem Vorrichtungsaspekt durch eine Katheteranordnung mit den Merkmalen des Anspruchs 1 und in ihrem Verfahrensaspekt durch ein Verfahren mit den Merkmalen des Anspruchs 13 gelöst. Zweckmäßige Fortbildungen des Erfindungsgedankens sind Gegenstand der jeweiligen abhängigen Ansprüche. Beansprucht werden auch ein Katheter sowie eine Flüssigkeitszuführeinheit sowie eine Druckauswertungseinheit zur Bildung einer Katheteranordnung gemäß Anspruch 1 sowie Ausgestaltungen jener Anordnungskomponenten.

Die Erfindung schließt die Überlegung ein, eine Katheteranordnung anzugeben, bei der sich die Kontaktkraft zwischen einer Katheterspitze und dem daran anliegenden Gewebe bestimmen lässt und die eine sehr niedrige Komplexität aufweist um kosteneffizient und maschinell hergestellt werden zu können. Dabei soll vollständig auf optische oder elektronische Sensorik, welche im Katheter angebracht ist, verzichtet werden.

Die für die Kraftmessung erforderliche messtechnische Sensorik soll, gemäß einem weiteren Aspekt der Erfindung, hauptsächlich in einem externen Gerät untergebracht sein, welches nicht direkt mit dem Patient in Kontakt gebracht wird und somit wiederverwendet werden kann. Zudem soll die Lösung eine weitestgehend automatisierte Herstellung durch die Verwendung einfacher Bauteile ermöglichen.

Hieraus ergibt sich der Aufbau der erfindungsgemäßen Anordnung, welche insbesondere einen als Einmal- bzw. Wegwerfartikel gefertigten Katheter und als weitere Komponenten eine über längere Zeiträume einsetzbare Flüssigkeitszuführeinheit und Auswertungseinheit umfasst. Mit der Erfindung lassen sich wesentliche Vorteile gegenüber bekannten Systemen realisieren, insbesondere die folgenden:

- Es gibt eine hohe Kostenersparnis, da auf teure optische und elektronische Messtechnik im Bereich der Einwegkomponenten (Katheter) eines derartigen Katheter Systems verzichtet werden kann.

- Das System wird umweltfreundlicher

- Das System lässt sich maschinell herstellen, da es im Katheter über ein Kanalsystem verfügt, welches mittels eines Extrusionsverfahrens äußerst kostengünstig hergestellt werden kann. Hier steckt die Komplexität ausschließlich in der einmaligen Ausgestaltung des Werkzeuges. Zudem können die Austrittsdüsen beispielsweise mittel Laserbearbeitung äußerst genau und kostengünstig hergestellt werden.

- Das System verzichtet vollständig auf teure optische Messtechnik, wie beispielsweise bei den FBG-Sensoren wo die Sensoren spektroskopisch ausgelesen werden.

- Die Erfindung ermöglicht es, eine Katheteranordnung bereitzustellen, welche von den Kosten kaum teurer ist als bisherige Katheter ohne Kraftmessfunktionalität womit sich ein enormer Marktvorteil für die Zukunft ergeben könnte.

Die Erfindung beruht auf dem Konzept einer Kraftmessung mittels hydrodynamischer Flussmessung einer durch eine Düse strömenden Flüssigkeit, wobei die Düse so ausgeführt ist, dass sie einen Öffnungsquerschnitt aufweist, welcher von einer externen Kraft abhängig ist. Bei der Flüssigkeit kann in einer besonders geeigneten Ausführung direkt die Kühlflüssigkeit verwendet werden.

Zur Erläuterung der Brauchbarkeit dieses Konzepts wird ausgegangen vom Gesetz von Hagen-Poiseuille:

$$j = \frac{\pi r^4 \Delta P}{8\eta l}$$

j    [m^3/s]
r    [m]
l    [m]
P    [pascal]

Umrechnung des Flusses:
1m^3/s = 6 10^13 nl/min

Annahmen:

Katheterlänge L=2 m
Durchmesser Fluidkanal R=300 $\mu$m = 300 10^-6 m
Viskosität Wasser $\eta$ = 1
Druckdifferenz dP = 2 bar = 2 10^5 pascal
Düsenbohrung r = 10 $\mu$m = 10 * 10^-6 m
Länge der Düsenbohrung l= 100 $\mu$m = 100 * 10^-6 m
Messgrenze für Flussmessungen eines handelsüblichen Flusssensors: 0.5 nl/min

Um die Funktionsweise des hier beschriebenen Kraftmesssensors zu gewährleisten, muss in erster Linie sichergestellt sein, dass die Änderung des Flusses hauptsächlich durch eine Variationen des Düsendurchmessers induziert wird und nicht etwa durch andere Einflüsse, wie beispielsweise eine Änderung des Schlauchquerschnittes. Es muss also erreicht werden, dass die Düse den größten Widerstand ausmacht. Dies bedeutet, dass der größte Druckabfall über dem Düsenbereich erfolgen muss und nicht über der Zuführung der Flüssigkeit zur Düse.

Um diesen Sachverhalt zu verdeutlichen, empfiehlt es sich, den Flüssigkeitsweg in zwei Abschnitte zu unterteilen: das Schlauchsystem (Länge L=2m, D=0.5mm) welches die Flüssigkeit zur Düse leitet, sowie den Düsenabschnitt selbst (l=200$\mu$m, d=20$\mu$m).

Nach dem hydrodynamischen Kontinuitätsprinzip muss der Fluss j überall im System gleich sein. Somit lässt sich nach dem Hagen Poisenilleschen Prinzip die Druckverteilung über die beiden Abschnitte wie folgt berechnen:

$$j = \frac{\pi r_{duese}^4 \Delta P_{duese}}{8\eta l_{duese}}$$

$$j = \frac{\pi r_{schlauch}^4 \Delta P_{schlauch}}{8\eta l_{schlauch}}$$

Gleichsetzen der beiden Gleichungen ergibt:

$$\frac{\Delta P_{duese}}{\Delta P_{schlauch}} = \frac{r_{schlauch}^4 l_{duese}}{r_{duese}^4 l_{schlauch}} = \frac{(300\mu m)^4 200\mu m}{(10\mu m)^4 2m} = 81$$

Das bedeutet, dass der Druckabfall bei den angenommenen Werten, welche so gewählt wurden, dass sie einer Katheterkonstruktion gerecht würden, an der Düse 81 mal grösser ist als der, welcher sich über das Schlauchsystem ergibt.

Ferner muss erreicht werden, dass der Dynamikbereich des Flusssensors vernünftig ausgenützt wird. Wir nehmen dafür an, dass der Druckabfall über der Düse 2 bar sein soll. Für den Fluss, welcher sich bei einer Druckdifferenz von 2 bar an der Düse gemäß den oben angegebenen Werten ergibt, gilt:

$$j = \frac{\pi r^4 \Delta P_{duese}}{8\eta l_{duese}} = \frac{\pi (10\mu m)^4 2 \cdot 10^5 pascal}{8 \cdot 1 \cdot 200\mu m} = 3.92 \cdot 10^{-12} m^3/s = 236 nl/min$$

Verglichen mit der Messgenauigkeit des Sensors von 0.5 nl/min zeigt dieser Vergleich, dass sich damit der Fluss sehr gut bestimmen lassen würde.

Abschließend soll noch demonstriert werden, wie der Fluss durch eine Veränderung von 10% des Düsenquerschnitts sich verändern würde.

Nimmt also der Querschnitt auf 0.9 des Ursprungswertes ab, so ergibt sich für den Fluss eine Abnahme von 0.9^4 = 0.65. Dies bedeutet, dass anstatt der ursprünglichen 236 nl/min nur noch 155 nl/ min ausströmen würden. Eine Änderung die messtechnisch sehr gut mit dem Sensor, welcher eine Auflösung von 0.5 nl/min hat, erfasst werden könnte!

Generell wäre es aber auch denkbar, mit höheren Flussraten bis 1 ml/min zu arbeiten. Bei den niedrigen Flussraten könnte sich nämlich die Längenänderung des Gesamtschlauches negativ auswirken. Wird dieser durch den Zugdraht beispielsweise gebogen, so würde auch eine Komprimierung des Volumens stattfinden. Eine grobe Abschätzung hat ergeben, dass die dadurch entstehende Flussrate ca. 1 µl/min ausmachen würde. Bei gemessenen Flüssen von 236 nl/min wäre dieser Einfluss schon erheblich und würde die Messung empfindlich stören.

In einer Ausführung der Erfindung weist der Katheter mindestens drei, insbesondere in gleichen Winkelabständen zueinander angeordnete, Fluidkanäle mit je einem proximalen Fluidanschluss und je einer distalen Düse mit veränderlichem Querschnitt und die Flüssigkeitszuführeinheit drei Flusssensoren auf, von denen jeder einem der drei mit distalen Düsen versehenen Fluidkanäle zugeordnet ist, und die Druckauswertungseinheit ist dreikanalig zur Auswertung der Signale der drei Flusssensoren zur Berechnung eines Kraftvektors der auf das distale Katheterende einwirkenden Kraft ausgebildet. In einer Ausgestaltung dieser Ausführung weisen die drei mit zugeordneten Flusssensoren versehenen Fluidkanäle gleichen Querschnitt und die an deren distalem Ende vorgesehenen Düsen gleiche Düsenform auf.

In einer weiteren Ausgestaltung ist die oder jede Düse benachbart vom äußersten distalen Ende des Katheters angeordnet, und in der Umgebung der Düse ist ein Katheterabschnitt aus elastisch verformbarem Material derart angeordnet, dass eine Krafteinwirkung auf das äußerste distale Ende zu einer proportionalen Verringerung des Düsenquerschnitts führt. In einer Ausgestaltung ist die oder jede Düse schlitzförmig mit zur Längsachse des Katheters im Wesentlichen senkrechter Erstreckung ausgeführt.

In einer praktisch wichtigen Ausführung der Anordnung ist der Katheter als Ablationskatheter mit mindestens einer Ablationselektrode und der oder jeder Fluidkanal als Spülkanal ausgebildet und die an seinem distalen Ende vorgesehene Düse benachbart zur Ablationselektrode angeordnet.

Zweckmäßigerweise sind in der Druckauswertungseinheit ein Verformungscharakteristika-Speicher zur Speicherung eines Wertesatzes von Verformungscharakteristika der oder jeder Düse und eine Verarbeitungskomponente vorgesehen, in der ein Auswertungsalgorithmus zur Bestimmung der einwirkenden Kraft aus dem gespeicherten Satz von Verformungscharakteristika implementiert ist.

In einer Ausgestaltung des vorgeschlagenen Verfahrens, unter Einsatz eines Katheters, mit mindestens drei, insbesondere in gleichen Winkelabständen zueinander angeordneten, Fluidkanälen mit je einem proximalen Fluidanschluss und je einer distalen Düse mit veränderlichem Querschnitt ein Kraftvektor der einwirkenden Kraft bestimmt wird, ist vorgesehen, dass eine Flüssigkeitszuführeinheit mit drei jeweils einem Fluidkanal zugeordneten Flusssensoren angeschlossen ist und in einer eingangsseitig signalmäßig mit den Flusssensoren verbundenen dreikanaligen Druckauswertungseinheit die Signale der drei Flusssensoren zur Berechnung des Kraftvektors benutzt werden.

Vorteile und Zweckmäßigkeiten der Erfindung ergeben sich im Übrigen aus der nachfolgenden Beschreibung von Ausführungsbeispielen anhand der Figuren. Von diesen zeigen:

Fig. 1        eine skizzenartige Darstellung einer Katheteranordnung gemäß einer Ausführungsform der Erfindung,

Fig. 2        eine skizzenartige Darstellung des distalen Katheterendes in Kontakt mit einer eine Kraft ausübenden Organ- bzw. Gefäßwandung und

Fig. 3A und 3B    eine Außenansicht und teilweise geschnittene Ansicht eines distalen Katheterendes einer anderen Ausführungsform.

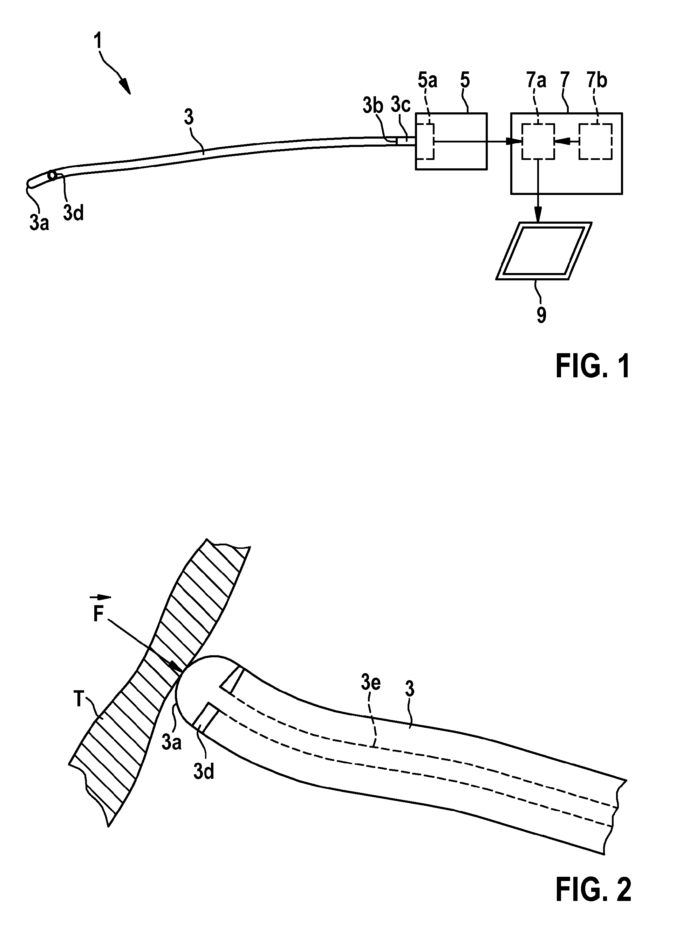

Fig. 1 zeigt schematisch eine Katheteranordnung 1, die einen schlauchartigen Katheter 3 mit einem distalen Ende 3a und einem proximalen Ende 3b umfasst, an dessen proximalem Ende ein Fluidanschluss 3c und nahe dessen distalem Ende eine Düsenöffnung 3d angeordnet sind. An den Fluidanschluss 3c des Katheters 3 ist eine Flüssigkeitszufuhreinheit 5 mit einem grundsätzlich bekannten Aufbau angeschlossen, wie er bei entsprechenden Einheiten von gespülten Ablationskathetern üblich ist. Eine Besonderheit der Flüssigkeitszufuhreinheit 5 besteht im Vorhandensein eines dem Fluidanschluss 3c des Katheters 3 zugeordneten Flusssensors 5a, mit dem ein Druckabfall in einem (hier nicht dargestellten) Fluidkanal im Katheter 3 quantitativ erfasst werden kann.

Der Flusssensor 5a steht signalmäßig in Verbindung mit einer Druckauswertungseinheit 7, und zwar speziell mit einer Verarbeitungskomponente 7a derselben. Der Verarbeitungskomponente 7a, in der ein Auswertungsalgorithmus zur Verarbeitung von mittels des Flusssensors 5a erfassten Druckabfalls-Werten implementiert ist, ist eine Speichereinheit 7b zugeordnet, in der auswertungsrelevante Charakteristika (Parameter) des Katheters 3 gespeichert sind. Der Ausgang der Verarbeitungskomponente 7a ist mit einer Anzeigeeinheit 9 verbunden, auf der aus den Druckabfalls-Werten berechnete Werte (Betragswerte oder Vektorkomponenten) einer auf das distale Katheterende 3a einwirkenden äußeren Kraft angezeigt werden können.

Fig. 2 zeigt eine Anwendungssituation des Katheters 3 mit der in kleinem Abstand vom distalen Ende 3a angeordneten Düse 3d in vergrößerter Darstellung. Hier ist auch der oben erwähnte, aber in Fig. 1 nicht dargestellte Fluidkanal 3e gezeigt, der vom proximalen Fluidanschluss 3c bis zur Düse 3d verläuft. In Kontakt des Katheterendes 3a mit einer Organ- oder Gefäßwandung T wird von dieser auf das Katheterende eine Kraft F ausgeübt. Bei entsprechend formelastischer Ausführung des distalen Katheterabschnitts führt diese Krafteinwirkung zu einer Verformung, und zwar speziell einer abschnittsweisen Querschnittsverringerung, der Düse 3d. Diese wiederum führt, wie weiter oben genauer erläutert, zu einer Druckänderung im Fluidsystem der Katheteranordnung, die mittels des Flusssensors 5a detektiert werden kann.

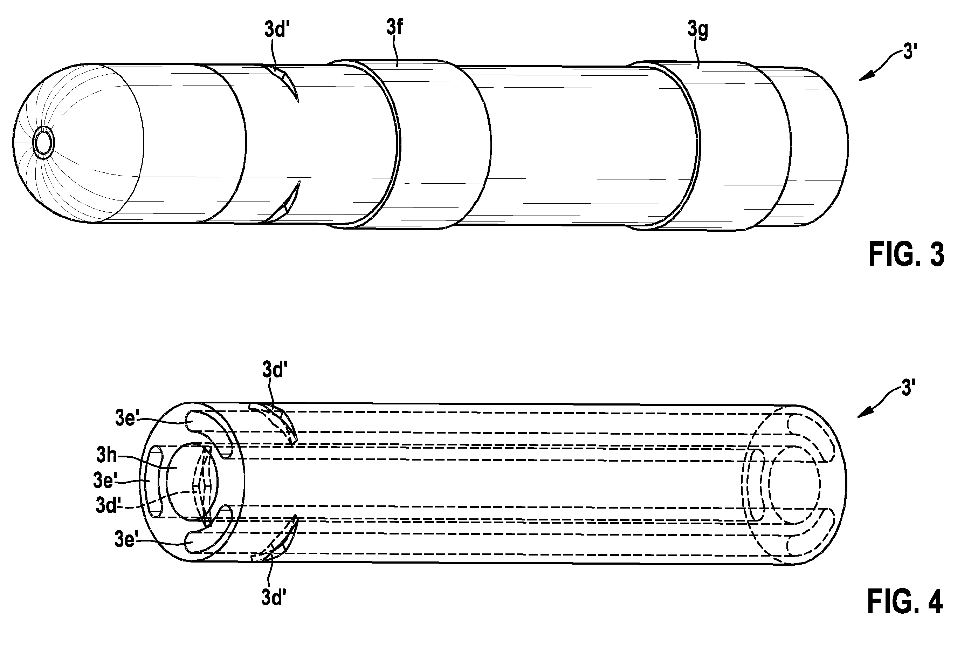

Fig. 3A und 3B zeigen das distale Ende eines modifizierten Katheters 3', der mit zwei voneinander beabstandet im distalen Endbereich angeordneten Ablationselektroden (Ringelektroden) 3f, 3g ausgestattet ist. Neben einem zentralen Lumen 3h, in dem in üblicher Weise Teile untergebracht sein können, die für die Ausführung der vorliegenden Erfindung nicht von Bedeutung sind, sind im Katheterkörper in gleichen Winkelabständen drei Fluidkanäle 3e' angeordnet, denen jeweils eine Austrittsdüse 3d' sowie (hier nicht dargestellt) am proximalen Katheterende jeweils ein Fluidanschluss zugeordnet sind. Distal von den schlitzförmigen Düsen 3d' sind die Fluidkanäle 3d' jeweils verschlossen, so dass vom proximalen Ende in die Fluidkanäle eingeleitete Flüssigkeit vollständig über die Düsen austreten muss.

Bei einer Kraft, welche direkt von vorne auf das System einwirkt, würden alle Düsen gestaucht. Wirkt dagegen eine Kraft aus seitlicher Richtung auf das System, so würde dies eine asymmetrische Stauchung bzw. Dehnung verursachen. Anhand einer Abbildungsmatrix, ließe sich damit aus den drei Sensorwerten der Kraftvektor, welcher auf das System wirkt berechnen. Die Fluidkanäle 3e' haben übereinstimmenden Querschnitt, und auch die Düsen 3d' haben die gleiche Form, so dass in einem von äußeren Krafteinwirkungen freien Zustand des Katheters in allen Fluidkanälen die gleichen Druckverhältnisse herrschen. Unter Einwirkung einer äußeren Kraft verformen sich die Düsen entsprechend den Vektorkomponenten dieser Kraft, so dass die in den einzelnen Fluidkanälen (über jeweils einen zugeordneten Flusssensor) erfassten Druckabfalls-Werte einen Rückschluss auf die Vektorkomponenten und somit letztlich eine näherungsweise vektorielle Bestimmung der einwirkenden Kraft erlauben.

Die Ausführung der Erfindung ist nicht auf die oben beschriebenen Beispiele und hervorgehobenen Aspekte beschränkt, sondern kann ebenso in einer Vielzahl von Abwandlungen erfolgen, die im Rahmen fachmännischen Handelns liegen.

**Patentansprüche**

1. Katheteranordnung (1), welche umfasst:

   einen Katheter (3; 3') mit einem proximalen (3b) und einem distalen Ende (3c) und mindestens einem durchgehenden Fluidkanal (3e; 3e'), an dessen proximalem Ende ein Fluidanschluss (3c) und an dessen distalem Ende eine Düse (3d; 3d') angeordnet ist, die einen durch eine auf das distale Katheterende einwirkende Kraft veränderlichen Querschnitt hat,
   eine mit dem proximalen Fluidanschluss des Katheters verbundene Flüssigkeitszuführeinheit (5) zur Zuführung von Flüssigkeit in den oder mindestens einen Fluidkanal des Katheters unter vorbestimmten Betriebsdruck, wobei in der Flüssigkeitszuführeinheit ein Flusssensor (5a) zur Erfassung eines Druckabfalls am von der Flüssigkeit durchströmten Fluidkanal im Betrieb der Katheteranordnung vorgesehen ist, und
   eine eingangsseitig signalmäßig mit dem Flusssensor verbundene Druckauswertungseinheit (7) zur Bestimmung einer auf das distale Katheterende des Katheters einwirkenden Kraft aus dem erfassten Druckabfall am Fluidkanal und vorbestimmten Verformungscharakteristika der zugeordneten Düse.

2. Katheteranordnung nach Anspruch 1, wobei der Katheter (3') mindestens drei, insbesondere in gleichen Winkelabständen zueinander angeordnete, Fluidkanäle (3e') mit je einem proximalen Fluidanschluss (3c) und je einer distalen Düse (3d') mit veränderlichem Querschnitt und die Flüssigkeitszuführeinheit drei Flusssensoren aufweist, von denen jeder einem der drei mit distalen Düsen versehenen Fluidkanäle zugeordnet ist, und die Druckauswertungseinheit dreikanalig zur Auswertung der Signale der drei Flusssensoren zur Berechnung eines Kraftvektors der auf das distale Katheterende einwirkenden Kraft ausgebildet ist.

3. Katheteranordnung nach Anspruch 2, wobei die drei mit zugeordneten Flusssensoren versehenen Fluidkanäle (3e') gleichen Querschnitt und die an deren distalem Ende vorgesehenen Düsen (3d') gleiche Düsenform aufweisen.

4. Katheteranordnung nach einem der vorangehenden Ansprüche, wobei die oder jede Düse (3d; 3d') benachbart vom äußersten distalen Ende (3a) des Katheters (3; 3') angeordnet ist und in der Umgebung der Düse ein Katheterabschnitt aus elastisch verformbarem Material derart angeordnet ist, dass eine Krafteinwirkung auf das äußerste distale Ende zu einer proportionalen Verringerung des Düsenquerschnitts führt.

5. Katheteranordnung nach Anspruch 4, wobei die oder jede Düse (3d; 3d') schlitzförmig mit zur Längsachse des Katheters (3; 3') senkrechter Erstreckung ausgeführt ist.

6. Katheteranordnung nach einem der vorangehenden Ansprüche, wobei der Katheter als Ablationskatheter (3') mit mindestens einer Ablationselektrode (3f, 3g) ausgebildet und der oder jeder Fluidkanal (3e') als Spülkanal ausgebildet und die an seinem distalen Ende vorgesehene Düse (3d') benachbart zu der oder einer Ablationselektrode angeordnet ist.

7. Katheteranordnung nach einem der vorangehenden Ansprüche, wobei in der Druckauswertungseinheit (7) ein Verformungscharakteristika-Speicher (7b) zur Speicherung eines Wertesatzes von Verformungscharakteristika der oder jeder Düse und eine Verarbeitungskomponente (7a) vorgesehen sind, in der ein Auswertungsalgorithmus zur Bestimmung der einwirkenden Kraft aus dem gespeicherten Satz von Verformungscharakteristika implementiert ist.

8. Katheter (3; 3') zur Bildung einer Katheteranordnung (1) nach einem der vorangehenden Ansprüche, mit einem proximalen (3b) und einem distalen Ende (3a) und mindestens einem durchgehenden Fluidkanal (3e; 3e'), an dessen proximalem Ende ein Fluidanschluss (3c) und an dessen distalem Ende eine Düse (3d; 3d') angeordnet ist, die einen durch eine auf das distale Katheterende einwirkende Kraft veränderlichen Querschnitt hat.

9. Katheter nach Anspruch 8, ausgebildet als Ablationskatheter (3') mit mindestens einer Ablationselektrode (3f, 3g), wobei der oder jeder Fluidkanal (3e') als Spülkanal ausgebildet und die an seinem distalen Ende vorgesehene Düse (3d') benachbart zu der oder einer Ablationselektrode angeordnet ist.

10. Flüssigkeitszuführeinheit (5) zur Bildung einer Katheteranordnung (1) nach einem der Ansprüche 1 bis 7, zur Zuführung von Flüssigkeit in den oder mindestens einen Fluidkanal des Katheters (3; 3') unter vorbestimmten Betriebsdruck, wobei in der Flüssigkeitszuführeinheit mindestens ein Flusssensor (5a) zur Erfassung eines Druckabfalls am von der Flüssigkeit durchströmten Fluidkanal im Betrieb der Katheteranordnung vorgesehen ist.

11. Druckauswertungseinheit (7) zur Bildung einer Katheteranordnung (1) nach einem der Ansprüche 1 bis 7, zur Bestimmung einer auf das distale Katheterende des Katheters (3; 3') einwirkenden Kraft aus dem erfassten Druckabfall am Fluidkanal (3e; 3e') und vorbestimmten Verformungscharakteristika der zugeordneten Düse (3d; 3d').

12. Druckauswertungseinheit (7) nach Anspruch 11, in der ein Verformungscharakteristika-Speicher (7b) zur Speicherung eines Wertesatzes von Verformungscharakteristika der oder jeder Düse (3d; 3d') und eine Verarbeitungskomponente (7a) vorgesehen sind, in der ein Auswertungsalgorithmus zur Bestimmung der einwirkenden Kraft aus dem gespeicherten Satz von Verformungscharakteristika implementiert ist.

13. Verfahren zur Bestimmung einer auf das distale Ende eines Katheters (3; 3') mit einem proximalen (3b) und einem distalen Ende (3a) und mindestens einem durchgehenden Fluidkanal (3e; 3e'), an dessen proximalem Ende ein Fluidanschluss (3c) und an dessen distalem Ende eine Fluiddüse (3d; 3d') angeordnet ist, die einen durch eine auf das distale Katheterende einwirkende Kraft veränderlichen Querschnitt hat, einwirkenden Kraft, wobei an den proximalen Fluidanschluss des Katheters eine Flüssigkeitszuführeinheit (5) zur Zuführung von Flüssigkeit in den Fluidkanal mit einem vorbestimmten Betriebsdruck angeschlossen wird, über einen Flusssensor (5a) in oder an der Flüssigkeitszuführeinheit ein Druckabfall am Fluidkanal im Betrieb des Katheters erfasst wird und in einer eingangsseitig signalmäßig mit dem Flusssensor verbundenen Druckauswertungseinheit (7) die einwirkende Kraft aus dem erfassten Druckabfall und Verformungscharakteristika der Düse bestimmt wird.

14. Verfahren nach Anspruch 13, wobei an einem Katheter (3') mit mindestens drei, insbesondere in gleichen Winkelabständen zueinander angeordneten, Fluidkanälen (3e') mit je einem proximalen Fluidanschluss und je einer distalen Düse (3d') mit veränderlichem Querschnitt ein Kraftvektor der einwirkenden Kraft bestimmt wird, indem eine Flüssigkeitszuführeinheit (5) mit drei jeweils einem Fluidkanal zugeordneten Flusssensoren (5a) angeschlossen und in einer eingangsseitig signalmäßig mit den Flusssensoren verbundenen dreikanaligen Druckauswertungseinheit die Signale der drei Flusssensoren zur Berechnung des Kraftvektors benutzt werden.

**FIG. 1**

**FIG. 2**

**FIG. 3**

3'

3g

3f

3d'

**FIG. 4**

3'

3d'

3d'

3e'

3h

3e'

3d'

3e'

Europäisches
Patentamt

European
Patent Office

Office européen
des brevets

## EUROPÄISCHER RECHERCHENBERICHT

Nummer der Anmeldung

EP 14 17 7423

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2013/172784 A1 (KIRSCHENMAN MARK B [US]) 4. Juli 2013 (2013-07-04) * Absätze [0030] - [0035]; Abbildungen 1-7 * ----- | 1-14 | INV. A61B18/14 ADD. A61B19/00 |
| A | EP 2 604 209 A2 (VASCOMED GMBH [DE]) 19. Juni 2013 (2013-06-19) * Absätze [0009] - [0019]; Abbildungen 3A-3C * ----- | 1-14 | |
| A | US 2008/161794 A1 (WANG HUISUN [US] ET AL) 3. Juli 2008 (2008-07-03) * Absatz [0076]; Abbildung 3 * ----- | 1-14 | |

RECHERCHIERTE SACHGEBIETE (IPC)

A61B

Der vorliegende Recherchenbericht wurde für alle Patentansprüche erstellt

| Recherchenort | Abschlußdatum der Recherche | Prüfer |
|---|---|---|
| Den Haag | 11. Februar 2015 | Mayer-Martenson, E |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
....................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                    EP 14 17 7423

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-02-2015

| Im Recherchenbericht angeführtes Patentdokument | Datum der Veröffentlichung | Mitglied(er) der Patentfamilie | Datum der Veröffentlichung |
|---|---|---|---|
| US 2013172784 A1 | 04-07-2013 | KEINE | |
| EP 2604209 A2 | 19-06-2013 | EP 2604209 A2<br>US 2013150805 A1 | 19-06-2013<br>13-06-2013 |
| US 2008161794 A1 | 03-07-2008 | US 2008161794 A1<br>WO 2008083003 A2 | 03-07-2008<br>10-07-2008 |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20080285909 A1
- WO 2009138957 A2
- US 5399854 A
- WO 2009007857 A2
- WO 2008003307 A2
- US 61446053 B
- US 61703272 B

**In der Beschreibung aufgeführte Nicht-Patentliteratur**

- **A. OTHONOS ; K. KALLI.** Fiber Bragg Gratings: Fundamentels and Applications in Telecommunications and Sensing. Artec House, 1999